(19) Europäisches Patentamt
European Patent Office
Office européen des brevets

(11) EP 2 258 473 A1

(12) EUROPEAN PATENT APPLICATION
published in accordance with Art. 153(4) EPC

(43) Date of publication:
08.12.2010 Bulletin 2010/49

(51) Int Cl.:
B01J 23/66 (2006.01)   B01J 35/02 (2006.01)
B01J 35/10 (2006.01)   C07D 301/08 (2006.01)
C07D 303/00 (2006.01)

(21) Application number: 09724212.7

(22) Date of filing: 18.03.2009

(86) International application number:
PCT/JP2009/055323

(87) International publication number:
WO 2009/119416 (01.10.2009 Gazette 2009/40)

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(30) Priority: 26.03.2008   JP 2008081548

(71) Applicant: Nippon Shokubai Co., Ltd.
Osaka-shi, Osaka 541-0043 (JP)

(72) Inventors:
• HIROTA, Hiroyuki
  Kawasaki-shi
  Kanagawa 210-0865 (JP)
• MIKAWA, Masatsugu
  Kawasaki-shi
  Kanagawa 210-0865 (JP)

(74) Representative: Barrett, Jennifer Catherine et al
Kilburn & Strode LLP
20 Red Lion Street
London WC1R 4PJ (GB)

(54) CATALYST FOR PRODUCTION OF ETHYLENE OXIDE, AND PROCESS FOR PRODUCTION OF ETHYLENE OXIDE USING THE CATALYST

(57) [PROBLEM]
A catalyst that enables to produce ethylene oxide in high efficiency and high selectivity, and a method for the production of ethylene oxide using the catalyst are provided.

[TECHNICAL SOLUTION]
A catalyst for the production of ethylene oxide comprising a catalyst component supported on a carrier comprising α-alumina as a main component, having a crush strength of at least 40 N, a specific surface area of at least 1.0 $m^2$/g, and a geometrical shape of a hollow cylinder, characterized in that the catalyst is used by packing in a reaction tube with an inner diameter of at least 28 mm, of a reactor for producing ethylene oxide; and a length/outer diameter ratio in the hollow cylinder is from 0.5 to 1.5, and an inner diameter/outer diameter ratio in the hollow cylinder is within a range of from 0.38 to 0.80. is provided.

EP 2 258 473 A1

**Description**

TECHNICAL FIELD

This invention relates to a catalyst for the production of ethylene oxide and a method for the production of ethylene oxide using the catalyst. In more detail, this invention relates to a catalyst which is superior in selectivity of ethylene oxide and is capable of producing ethylene oxide in high selectivity, and a method for the production of ethylene oxide using this catalyst.

BACKGROUND ART

It has been widely performed industrially to produce ethylene oxide by vapor phase oxidation of ethylene with a molecular oxygen-containing gas in the presence of a silver catalyst. As for the silver catalyst to be used in this catalytic vapor phase oxidation, many technologies have been proposed on a carrier thereof, a supporting method, and a reaction accelerator or the like.

Catalytic activity, selectivity and catalyst life of the silver catalyst have reached a high level already, however, still more enhancement of catalytic performance thereof has been required. For example, as for selectivity, because of a large production scale of ethylene oxide, even only 1% of enhancement of selectivity provides extreme saving of use amount of ethylene as raw material, and economical effect thereof is large. Under such circumstance, development of the silver catalyst having more superior catalytic performance has been a continuous theme of researchers in the relevant technical field.

For example, JP-T-2007-500596 discloses a catalyst having silver supported on a carrier having $\alpha$-alumina as a main component. As described in JP-T-2007-500596, as the catalyst for the production of ethylene oxide, one having hollow cylinder shape is used. In particular, it has been known that one having an inner diameter/outer diameter ratio of up to 0.3 is suitable.

DISCLOSURE OF INVENTION

PROBLEMS TO BE SOLVED BY THE INVENTION

However, there was a problem that catalytic performance by the silver catalyst described in JP-T-2007-500596 is still insufficient.

Accordingly, it is an object of this invention to provide a catalyst which is capable of producing ethylene oxide in high efficiency and high selectivity, and a method for the production of ethylene oxide using this catalyst.

MEANS FOR SOLVING THE PROBLEMS

We have intensively studied a way to solve the above-describedproblem. As a result, we found that a catalyst for the production of ethylene oxide, which is capable of producing ethylene oxide in high efficiency and high selectivity, can be provided, when using a carrier with large surface area, in particular, a carrier with a specific surface area of at least 1.0 $m^2$/g, by increasing the inner diameter/outer diameter ratio of the hollow cylinder more than one conventionally used, and have thus completed this invention.

That is, this invention is a catalyst for the production of ethylene oxide comprising a catalyst component supported on a carrier comprising $\alpha$-alumina as a main component, having a crush strength of at least 40 N, a specific surface area of at least 1.0$m^2$/g, and a geometrical shape of a hollow cylinder, **characterized in that** the catalyst is used by packing in a reaction tube with an inner diameter of at least 28 mm, of a reactor for producing ethylene oxide; and a length/outer diameter ratio in the hollow cylinder is from 0.5 to 1.5, and an inner diameter/outer diameter ratio in the hollow cylinder is within a range of from 0.38 to 0.80.

EFFECTS OF THE INVENTION

According to this invention, a catalyst for the production of ethylene oxide, which is capable of producing ethylene oxide in high efficiency and high selectivity, and a method for the production of ethylene oxide using this catalyst can be provided.

BEST MODE FOR CARRYING OUT THE INVENTION

Explanation will be given below on embodiments of this invention.

A first aspect of this invention is a catalyst for the production of ethylene oxide comprising a catalyst component supported on a carrier comprising α-alumina as a main component, having a crush strength of at least 40 N, a specific surface area of at least 1.0 m$^2$/g, and a geometrical shape of a hollow cylinder, **characterized in that** the catalyst is used by packing in a reaction tube with an inner diameter of at least 28 mm, of a reactor for producing ethylene oxide; and a length/outer diameter ratio in the hollow cylinder is from 0.5 to 1.5, and an inner diameter/outer diameter ratio in the hollow cylinder is within a range of from 0.38 to 0.80.

As described above, the carrier to be used for the catalyst for the production of ethylene oxide of this invention has a large value of a specific surface area of at least 1.0 m$^2$/g. The specific surface area of the carrier of equal to or higher than 1.0 m$^2$/g is capable of supporting necessary amount of a catalyst component. In addition, it increases number of fine pores sufficiently and makes high dispersion supporting of the catalyst component easy. Further, it increases area of the catalyst component, which is an active site of a catalytic reaction. The specific surface area of the carrier is preferably equal to or larger than 1.1 m$^2$/g, and more preferably equal to or larger than 1.2 m$^2$/g. The specific surface area of the carrier is preferably equal to or smaller than 5.0 m$^2$/g, and more preferably equal to or smaller than 3.0 m$^2$/g. The specific surface area within the above range is capable of maintaining pore diameter of the carrier to a certain large degree of value, and repressing sequential oxidation of ethylene oxide during production of ethylene oxide using a catalyst produced. It should be noted that, as the value of the specific surface area of the carrier, value obtained by a method described in Examples to be described later is adopted.

The carrier to be used in the catalyst for the production of ethylene oxide of this invention has a crush strength of at least 40 N. The crush strength of the carrier is preferably equal to or higher than 50 N, and more preferably equal to or higher than 60 N. The crush strength within the above range is capable of maintaining sufficient mechanical strength, even when the inner diameter/outer diameter ratio of the hollow cylinder is relatively large value. The case where the crush strength is lower than 40 N generates crack or powdering of the catalyst in catalyst in packing into the reaction tube, and increases pressure loss, which becomes disadvantageous in view of apparatus or utility. The upper limit value of the crush strength of the carrier is not especially limited. It should be noted that, as value of the crush strength of the carrier, value obtained by a method described in Examples to be described later is adopted.

The carrier to be used in the catalyst for the production of ethylene oxide of this invention has shape of a hollow cylinder, and a length/outer diameter ratio of from 0.5 to 1.5, and an inner diameter/outer diameter ratio of 0.38 to 0.80. Here, the hollow cylinder is not necessarily a complete cylinder, and it is enough to be substantially hollow cylinder shape. Preferably, the length/outer diameter ratio is 0.7 to 1.3, and more preferably 0.8 to 1.2. In addition, the inner diameter/outer diameter ratio is preferably 0.38 to 0.65, and more preferably 0.38 to 0.55. By using a catalyst with hollow cylinder shape having the length/outer diameter ratio and the inner diameter/outer diameter ratio within the above range, activity and selectivity of the catalyst enhance. In addition, life characteristics also enhance. The inner diameter/outer diameter ratio of smaller than 0.38 increases packing density and pressure loss, which becomes disadvantageous in view of apparatus or utility, and may incur a combustion reaction. On the other hand, the inner diameter/outer diameter ratio of larger than 0.80 decreases thickness of the catalyst, decreases mechanical strength of the catalyst and may incur difficulty of practical application as an industrial catalyst.

The length, outer diameter and inner diameter of the carrier are not especially limited, as long as they satisfy the above ratios, however, the length is preferably 1.5 to 30 mm, and more preferably 2.5 to 18 mm, and still more preferably 5.0 to 12 mm. The outer diameter of the carrier is preferably 3.0 to 20 mm, more preferably 5.0 to 12 mm. The inner diameter of the carrier is preferably 1.0 to 16 mm, an more preferably 2.0 to 8.0 mm.

In this description, values of the inner diameter, outer diameter and length of the carrier shall be determined by the following methods. Firstly, 20 carriers sampled by a random method are prepared. Next, burrs adhered at the inner diameter side of the carrier are removed with tweezers. As for the above 20 carriers, average value of maximal values and minimal values of the inner diameter measured with a caliper is adopted as the inner diameter value. In addition, average value of maximal values and minimal values of the outer diameter measured with a caliper is adopted as the outer diameter value. As the length, by measuring the length at one place by each carrier with a caliper, average value of 20 values is adopted as the length value.

Reason for the catalyst for the production of ethylene oxide of this invention using the above carrier to show high performance is not clear, however, increase in number of fine pores accompanying with increase in specific surface area is considered as one reason. It is considered that in the case of increased number of fine pores, shape with relatively small inner diameter/outer diameter ratio, as in a conventional one, lengthens residence time of ethylene oxide generated in the fine pores, resulting in progress of sequential oxidation and incurs decrease in selectivity.

The bulk density of the carrier is preferably 0.5 to 1.0 kg/L, more preferably 0.6 to 0.9 kg/L, and still more preferably 0.65 to 0.80 kg/L. The bulk density of the carrier within the above range is capable of providing a suitable packing density and thus producing a catalyst with sufficient strength. It should be noted that, as for value of the bulk density of the carrier, value obtained by a method described in Examples to be described later is adopted.

A composition of the carrier is not especially limited, as long as it comprises α-alumina as a main component. Here, the carrier "comprises α-alumina as a main component" means that it may contain partially alumina of different form other than α-alumina, such as, γ-alumina, amorphous alumina. Content of alumina in the carrier is preferably equal to or higher than 90% by mass, more preferably equal to or higher than 95% by mass, and still more preferably equal to or higher than 98% by mass relative to the total mass, 100% by mass, of the carrier. Other composition is not especially limited, as long as it is one comprising α-alumina as a main component, however, the carrier may include, for example, an oxide of an alkali metal or an alkaline earth metal, or an oxide of atransitionmetal. The content thereof is also not especially limited, however, the content of the oxide of the alkali metal or the alkaline earth metal is preferably 0 to 5% by mass, and more preferably 0.01 to 4% by mass, as converted to an oxide. In addition, the content of the oxide of the transition metal is preferably 0 to 5% by mass, and more preferably 0.01 to 3% by mass, as converted to an oxide.

In addition, the carrier usually contains silica (silicon dioxide). Content of silica in the carrier is not especially limited, however, preferably 0.01 to 10.0% by mass, more preferably 0.1 to 5.0% by mass, and still more preferably 0.2 to 3.0% by mass.

It should be noted that, the composition of the above carrier or the content of each component may be determined using X-ray fluorescence analysis method.

Particle diameter of an α-alumina as a raw material of the carrier, is not especially limited, however, a primary particle diameter of the α-alumina is preferably 0.01 to 100 μm, more preferably 0.1 to 20 μm, still more preferably 0.5 to 10 μm, and particularly preferably 1 to 5 μm. In addition, secondary particle diameter of the α-alumina is preferably 0.1 to 1,000 μm, more preferably 1 to 500 μm, still more preferably 10 to 200 μm, and particularly preferably 30 to 100 μm.

A pore volume of the carrier is also not especially limited, however, preferably 0.2 to 0.6 cm$^3$/g, more preferably 0.3 to 0.5 cm$^3$/g, and still more preferably 0.35 to 0.45 cm$^3$/g. The pore volume of the carrier of equal to or larger than 0.2 cm$^3$/g is preferable in view of making supporting of the catalyst component easy. On the other hand, the pore volume of the carrier of equal to or smaller than 0.6 cm$^3$/g is preferable in view of securing strength of the carrier in a practical level. It shouldbe noted that, as the value of the pore volume of the carrier, value obtained by Mercury Porosimetry is adopted.

A pore size which the carrier has is also not especially limited, however, a median pore diameter is preferably 0.1 to 10 μm, more preferably 0.2 to 4.0 μm, still more preferably 0.3 to 3.0 μm, and particularly preferably 0.4 to 1.5 μm. The median pore diameter of equal to or larger than 0.1 μm is capable of repressing sequential oxidation of ethylene oxide accompanying with residence of generated gas in producing ethylene oxide. On the other hand, the median pore diameter of equal to or smaller than 10 μm is capable of securing strength of the carrier in a practical level. It should be noted that, as the value of the median pore diameter, value obtained by Mercury Porosimetry is adopted.

A water absorption of the carrier is also not especially limited, however, preferably 10 to 70%, more preferably 20 to 60%, and still more preferably 30 to 50%. The water absorption of the carrier of equal to or higher than 10% makes supporting of the catalyst component easy. On the other hand, the water absorption of the carrier of equal to or lower than 70% is capable of securing strength of the carrier in a practical level. It should be noted that, as the value of the water absorption, value obtained by a method described in Examples to be described later is adopted.

The catalyst for the production of ethylene oxide of this invention comprises a catalyst component supported on the above carrier. Specific form of the catalyst component is not especially limited, and conventionally known knowledge may be referred as appropriate, however, it is preferable that silver is essentially contained as the catalyst component. In addition, a catalyst component to be used generally as a reaction accelerator may be supported on the carrier, other than silver. A typical example of the reaction accelerator includes an alkali metal, specifically lithium, sodium, potassium, rubidium and cesium or the like. Other than an alkali metal, thallium, sulfur, chromium, molybdenum, tungsten, rhenium or the like may also be used as the reaction accelerator. These reaction accelerators may be used alone as one kind, or two or more kinds may be used in combination. Among these, cesium may be used suitably as the reaction accelerator.

Supporting amount of silver or the reaction accelerator is not especially limited, and may be supported in an amount effective in producing ethylene oxide. For example, in the case of silver, the supporting amount thereof is 1 to 30% by mass, and preferably 5 to 20% by mass, based on mass of the catalyst for the production of ethylene oxide. In addition, the supporting amount of the reaction accelerator is usually 0.001 to 2% by mass, preferably 0.01 to 1% by mass, and more preferably 0.01 to 0.7% by mass, based on mass of the catalyst for the production of ethylene oxide. In particular, optimal supporting amount of the reaction accelerator is different depending on difference of property of the carrier or a combination of the reaction accelerators. Therefore, it is preferable that catalysts with different supporting amounts of the reaction accelerator are prepared in advance, and after performance evaluation of the relevant catalysts, the supporting amount of the reaction accelerator showing the highest performance is determined, and amount of the reaction accelerator showing such highest performance is supported, and thus the catalyst is prepared. It should be noted that, in the following Examples and Controls, the catalyst was prepared after determining the supporting amount of the reaction accelerator showing the highest performance in advance, in this way.

It should be noted that, shape of the catalyst depends on shape of the carrier to be used. That is, the catalyst of this invention has shape of a hollow cylinder. Values of the length, outer diameter and inner diameter of the hollow cylinder are substantially the same as those of the carrier to be used.

The reactor for producing ethylene oxide to be used in this invention may be a single-tubular reactor or a multi-tubular reactor, however, a multi-tubular reactor having multiple reaction tubes may be used preferably industrially. The catalyst for the production of ethylene oxide of this invention is packed in the reaction tube of the reactor for producing ethylene oxide in a packing density of at least 0.5 kg/L, more preferably 0.6 to 0.9 kg/L, and still more preferably 0.65 to 0.80 kg/L. The packing density of equal to or higher than 0.5 kg/L is capable of producing ethylene oxide in high efficiency and high selectivity. As the reaction tubes of the reactor for producing ethylene oxide, those with an inner diameter of at least 28 mm, preferably 28 to 50 mm, and more preferably 30 to 40 mm may be used. The case of the inner diameter of the reaction tube of below 28 mm increases number of the reaction tubes, and could increase manufacturing cost of the reactor. In addition, the inner diameter of the reaction tube of over 50 mm decreases heat removal efficiency and may increase heat accumulation in the catalyst layer. A packing method for the catalyst for the production of ethylene oxide in the reactor for producing ethylene oxide is also not especially limited. It should be noted that, as value of the packing density of the catalyst, value obtained by a method described in Examples to be described later is adopted.

In the reactor for producing ethylene oxide to be used in this invention, the catalyst for the production of ethylene oxide has a value of outer diameter or length, whichever is larger, of preferably 10 to 50%, more preferably 15 to 45%, and still more preferably 20 to 40%, relative to inner diameter of the reaction tube. When the value of outer diameter or length, whichever is larger, relative to inner diameter of the reaction tube, is smaller than 10%, packing density and pressure loss increase, which becomes disadvantageous in view of both apparatus and utility, and may incur a combustion reaction. On the other hand, when the value of outer diameter or length, whichever is larger, relative to inner diameter of the reaction tube, is larger than 50%, content of silver in the catalyst layer decreases and activity or life of the catalyst may decrease.

A content of silver as a catalyst component to be contained in the catalyst layer, is preferably 30 to 140 kg/m$^3$, more preferably 60 to 135 kg/m$^3$, and still more preferably 100 to 130 kg/m$^3$. The content of silver to be contained in the catalyst layer of higher than 140 kg/m$^3$ increases packing density and pressure loss, which may become disadvantageous in view of both apparatus and utility. On the other hand, the content of silver to be contained in the catalyst layer of lower than 30 kg/m$^3$ decreases content of silver in the catalyst layer and may decrease activity or life of the catalyst. As the value of the content of silver to be contained in the catalyst layer, value expressed by the following Expression is adopted.

$$\text{Content of silver in the catalyst layer } (kg/m^3) = [(\text{supporting amount of silver } (\% \text{ by mass})/100)) \times \text{packing density } (kg/L)] \times 1000$$

The catalyst for the production of ethylene oxide of this invention may be prepared according to a conventionally known production method.

As for a preparation method for the carrier, it has been known that by adopting the following preparation method, size or property of the carrier can be controlled. That is, 1) a method for adding a pore forming agent with a desired size and amount into a parent powder having $\alpha$-alumina as a main component, 2) a method for formulating at least two kinds of parent powders having different property in a desired mixing ratio, and 3) a method for calcining the carrier at desired temperature for desired time or the like, and a method by combining them has also been known. For example, to the $\alpha$-alumina, a molding auxiliary agent having effect for enhancing moldability, a reinforcing agent or a binder for enhancing strength of the catalyst, and a pore forming agent for forming fine pores to the catalyst are added and mixed. As a substance to be added, one not to give bad influence on catalytic performance by the addition is preferable. For example, an inorganic binder such as silica, alumina, silica-alumina, glass fiber, silicon carbide, silicon nitride, graphite may be added. An organic binder such as ethylene glycol, glycerin, propionic acid, maleic acid, benzyl alcohol, propyl alcohol, butyl alcohol, cellulose, methylcellulose, starch, polyvinyl alcohol or phenol is added as needed. In addition, a shell or a seed of a peach, an apricot, a chestnut or the like with uniform particle diameter or a substance with uniform particle diameter, which disappears by calcining, may be added as the pore forming agent. After sufficient mixing using a mixing machine such as a kneader by further adding water, it is molded into desired shape using a suitable mold by extrusion molding, granulated, dried and then calcined. These preparation methods are described, for example, in "Property of porous substances and application technology thereof" edited by Yoh Takeuchi, and issued by Fujitec Corporation (1999). In addition, JP-A-5-329368, JP-A-2001-62291, JP-A-2002-136868, JP-B-2983740, JP-B-3256237, JP-B-3295433 or the like may also be referred.

Explanation will be given below on one example of a method for producing a catalyst for the production of ethylene oxide of this invention using the above-described carrier, however, the technical scope of this invention should be determined based on description of claims, and should not be limited to only the following method.

Firstly, the carrier is prepared. Because a preparation method for the carrier is the same as described above, detailed explanation is omitted here.

Meanwhile, a solution for making silver supported on the carrier is prepared. Specifically, a silver compound alone, or with a complexing agent for forming a silver complex, or a reaction accelerator as needed, is added to a solvent such as water.

Here, a silver compound includes, for example, silver nitrate, silver carbonate, silver oxalate, silver acetate, silver propionate, silver lactate, silver citrate, silver neodecanoate or the like. In addition, a complexing agent includes, for example, monoethanol amine, diethanol amine, triethanol amine, ethylene diamine, propylene diamine or the like. These silver compounds or complexing agents maybe used alone as only one kind, or two or more kinds may be used in combination.

Next, the solution obtained as above is impregnated into the carrier prepared as above. In this case, the reaction accelerator may be dissolved in the solution in the pre-stage of the impregnation of the solution to the carrier, and impregnated at the same time, or may be supported after supporting silver.

Subsequently, it is dried and calcined. The drying is preferably performed in atmosphere of air, oxygen or inert gas (for example, nitrogen), at a temperature of 80 to 120°C. In addition, the calcining is preferably performed in atmosphere of air, oxygen, overheated steam, or inert gas (for example, nitrogen), at a temperature of 150 to 700°C, and preferably 200 to 600°C. It should be noted that, the calcining may be performed in one stage only or may be performed in two stages or more. Preferable calcining condition includes to perform the first stage calcining in air atmosphere at 150 to 250°C for 0.1 to 10 hours, and to perform the second stage calcining in air atmosphere at 250 to 450°C for 0.1 to 10 hours. Still more preferably, after such second stage calcining, still more the third stage calcining may be performed in inert gas atmosphere (for example, nitrogen, helium, argon or the like) at 450 to 700°C for 0.1 to 10 hours.

A second aspect of this invention is a method for the production of ethylene oxide using the catalyst for the production of ethylene oxide of the first aspect of this invention.

The method for the production of ethylene oxide, of the second aspect of this invention, is performed in accordance with a usual method, except that the catalyst for the production of ethylene oxide of the first aspect of this invention is used as a catalyst. Preferably, a method for catalytic vapor phase oxidation of ethylene with a molecular oxygen-containing gas such as air, oxygen and oxygen-enriched air is used.

For example, general condition in an industrial production scale, that is, a reaction temperature of 150 to 300°C, preferably 180 to 280°C, a reaction pressure of 2 to 40 kg/cm$^2$G, preferably 10 to 30 kg/cm$^2$G, a space velocity of 1, 000 to 30, 000 hr$^{-1}$(STP), preferably 3, 000 to 8, 000 hr$^{-1}$ (STP) is adopted. As raw material gas to be contacted with the catalyst, there is included one composed of 0.5 to 40% by volume of ethylene, 3 to 10% by volume of oxygen, 1 to 20% by volume of carbon dioxide gas and the balance of inert gas such as nitrogen, argon, steam, and a lower hydrocarbon such as methane, ethane, and still more contains as a halide as a reaction inhibitor such as ethylene dichloride, methyl chloride, ethyl chloride, vinyl chloride, diphenyl chloride or the like, in an amount of 0.1 to 10 ppm by volume.

EXAMPLES

Explanation will be given on effect of this invention with reference to the following Examples and Controls. However, the technical scope of this invention should not be limited to these Examples. It should be noted that, in this Examples, measurement of various kinds of parameters was performed by the following methods. As a reactor for producing ethylene oxide, a multi-tubular reactor is used industrially, however, in this Examples, performance evaluation was performed using a single-tubular reactor.

<Packing density of the reaction tube>

Packed mass of the catalyst and length of the catalyst packed layer, in packing the catalyst in a reaction tube having desired inner diameter, were measured to calculate the packing density by the following Expression 1.

```
        [Expression 1]

        Packing density (kg/L) = Packed mass of the catalyst

   (kg)/ packed volume of the catalyst (L)
```

<Bulk density of the carrier>

By packing the carrier into a 1 L measuring cylinder (an inner diameter of 66 mm) in a speed of 2 L/min up to a 1 L gauge line to measure the mass and calculate the bulk density by the following Expression 2.
$$[\text{Expression 2}]$$
$$\text{Bulk density (kg/L) = Packed mass of the carrier (kg)/ 1 (L)}$$

<Crush strength>

Using a precise competence measurement apparatus (manufactured by Marubishi Science Machine Manufacturing Co., Ltd.), crush strength from a lateral direction of the carrier (a vertical direction relative to the length) was measured, and average value of 50 values was adopted as the crush strength.

<Measurement of specific surface area of the carrier>

After pulverizing the carrier, about 0 . 2 g of a classified substance to a particle diameter of 0.85 to 1.2 mmwas precisely weighed. The weighed sample was deaerated at 200°C for at least 30 minutes to measure the specific surface area by the BET (Brunauer-Emmet-Teller) method.

<Measurement of water absorption of the carrier>

It was measured by the following method in accordance with a method described in JIS standard (JIS R 2205 (1998)).
a) The carrier before crushing was put in a drier maintained at 120 °C to weigh mass when constant mass was reached (dry mass: W1 (g)).
b) The carrier weighed in the above a) was immersed into water, and after boiling for equal to or longer than 30 minutes, it was cooled in water at room temperature to obtain a sample saturated with water.
c) The sample saturated with water obtained in the above b) was took out from water, wiped the surface quickly with a wet cloth, and after removing water droplets, mass was weighed (mass of the sample saturated with water: W2 (g)).
d) Using W1 and W2 obtained above, the water absorption was calculated according to the following Expression 3.
$$[\text{Expression 3}]$$
$$\text{Water absorption (\%) = [(W2-W1) / W1] x100}$$

<Wear rate of the carrier>

It was measured by the following method.
a) 100 mL of the carrier was put in a 300-mL conical beaker.
b) Pure water was added into the above conical beaker up to 250 mL.
c) The above b) was heated with an electric heater and boiled for 30 minutes.
d) After the boiling operation, the following operation was repeated five times: Pure water remained in the conical beaker was discharged, pure water was added newly, and only pure water was discarded.
e) Washing operation of the above b) to d) was repeated three times.
f) The carrier after washing was dried in a drier at 120°C over night.
g) The carrier after the drying was cooled to room temperature to perform weighing (mass before a test: W3 (g)).
h) The carrier obtained in the above g) was rotated in a ball mill made of stainless steel (an outer diameter of 90 mm, a height of 90 mm) in 106 ppm for 30 minutes.
i) Total amount of the carrier after the rotation was transferred to a sieve made of stainless steel (an inner diameter of 150 mm, a sieve mesh size of 1.7 mm) to weigh after that (mass after sieving: W4 (g)).
j) Using W3 and W4 obtained above, the wear rate was calculated according to the following Expression 4.

$$[\text{Expression 4}]$$
$$\text{Wear rate (\%)} = [(W3-W4)/W3] \times 100$$

(Example 1)

A carrier having α-alumina as a main component (a ring of 8.6 mm, bulk specific gravity: 0.74 g/mL, water absorption: 38.2%, content of alumina: 99.0% by mass) was prepared.

For 4 L of this carrier, boiling processing using 4-L of distilled water for equal to or longer than 30 minutes was repeated three times. After that, it was sufficiently dried in a drier maintained at 120°C to obtain a carrier A. The carrier A, as shown in Table 1, had hollow cylinder shape with an outer diameter of 8.57 mm, an inner diameter of 3.79 mm, and a length of 8.57 mm, and specific surface area was 1.54 $m^2$/g and crush strength was 64.0 N.

Meanwhile, into aqueous slurry containing 520 g of silver oxalate (water content in the aqueous slurry: 150 g), 100 mL of water and a solution dissolved with 3.3 g of cesium nitrate into 250 mL of water were added to make a mud-like substance. Then, 250 mL of ethylene diamine was added thereto and dissolved by sufficient stirring to prepare an impregnation solution.

The resultant impregnation solution was impregnated to 2000 g of the carrier A heated in advance at about 100°C. Then, it was subjected to heating, concentration and drying, and calcined in air flow at 400°C for 20 minutes to obtain a catalyst precursor.

The resultant catalyst precursor was packed in a sealing container made of stainless steel, which can be introduced inert gas from outside, and calcined in an electric furnace at a catalyst layer temperature of 530°C for 3 hours, under blowing nitrogen gas to prepare a catalyst for the production of ethylene oxide A.

(Example 2)

According to a similar method as in the above Example 1, except that a carrier B having shape and property shown in Table 1 was used and the addition amount of cesium nitrate was changed to 4.0 g, a catalyst for the production of ethylene oxide B was prepared.

(Example 3)

According to a similar method as in the above Example 1, except that a carrier C having shape and property shown in Table 1 was used and the addition amount of cesium nitrate was changed to 4.0 g, a catalyst for the production of ethylene oxide C was prepared.

(Example 4)

According to a similar method as in the above Example 1, except that a carrier D having shape and property shown in Table 1 was used and the addition amount of cesium nitrate was changed to 4.0 g, a catalyst for the production of ethylene oxide D was prepared.

(Example 5)

According to a similar method as in the above Example 1, except that a carrier E having shape and property shown in Table 1 was used and the addition amount of cesium nitrate was changed to 4.4 g, a catalyst for the production of ethylene oxide E was prepared.

(Control 1)

According to a similar method as in the above Example 1, except that a carrier "a" having shape and property shown in Table 1 was used and the addition amount of cesium nitrate was changed to 2.7 g, a catalyst for the production of ethylene oxide "a" was prepared.

(Control 2)

According to a similar method as in the above Example 1, except that a carrier "b" having shape and property shown in Table 1 was used and the addition amount of cesium nitrate was changed to 2.7 g, a catalyst for the production of ethylene oxide "b" was prepared.

(Control 3)

According to a similar method as in the above Example 1, except that a carrier "c" having shape and property shown in Table 1 was used and the addition amount of cesium nitrate was changed to 8.7 g, a catalyst for the production of ethylene oxide "c" was prepared.

(Control 4)

According to a similar method as in the above Example 1, except that a carrier "d" having shape and property shown in Table 1 was used and the addition amount of cesium nitrate was changed to 4.7 g, a catalyst for the production of ethylene oxide "d" was prepared.

(Control 5)

According to a similar method as in the above Example 1, except that a carrier "e" having shape and property shown in Table 1 was used and the addition amount of cesium nitrate was changed to 4.4 g, a catalyst for the production of ethylene oxide "e" was prepared.

(Control 6)

According to a similar method as in the above Example 1, except that a carrier "f" having shape and property shown in Table 1 was used and the addition amount of cesium nitrate was changed to 4.7 g, a catalyst for the production of ethylene oxide "f" was prepared.

(Control 7)

According to a similar method as in the above Example 1, except that a carrier "g" having shape and property shown in Table 1 was used and the addition amount of cesium nitrate was changed to 5.1 g, a catalyst for the production of ethylene oxide "g" was prepared.

(Control 8)

According to a similar method as in the above Example 1, except that a carrier "h" having shape and property shown in Table 1 was used and the addition amount of cesium nitrate was changed to 4.4 g, a catalyst for the production of ethylene oxide "h" was prepared.

(Control 9)

According to a similar method as in the above Example 1, except that a carrier "i" having shape and property shown in Table 1 was used and the addition amount of cesium nitrate was changed to 4.0 g, a catalyst for the production of ethylene oxide "i" was prepared.

(Control 10)

According to a similar method as in the above Example 1, except that a carrier "j" having shape and property shown in Table 1 was used and the addition amount of cesium nitrate was changed to 6. 5 g, a catalyst for the production of ethylene oxide "j" was prepared. However, the powdering of the carrier was confirmed in the impregnation step. In addition, because of generation of crack of the catalyst in packing the catalyst in the reactor, performance evaluation was not able to be performed.

<Measurement of conversion/selectivity of the catalyst>

Each of the catalysts A to E, and "a" to "j" obtained in Examples 1 to 5 and Controls 1 to 10 was packed in a reactor made of stainless steel, which was a double-tubular type, and a heating type of an exterior part, having an inner diameter of 35 mm and a tube length of 7500 mm, to form a packed layer. Then, mixed gas composed of 21% by volume of ethylene, 7.5% by volume of oxygen, 7% by volume of carbon dioxide and the balance of methane, argon, nitrogen, ethane (50% by volume of methane, 12% by volume of argon and 2.5% by volume of the balance (nitrogen and ethane)), and further containing 2.5 ppm of ethylene dichloride was introduced into the relevant packed layer to produce ethylene oxide under condition of a reaction pressure of 20 kg/cm$^2$G, and a space velocity of 6,000 hr$^{-1}$. Reaction temperature was set at condition described in the following Table 1. In addition, ethylene oxide was produced by a similar method as above, except that the catalyst A obtained in Example 1 was used and the inner diameter of the reaction tube was set at 31 mm, and it was referred to as Example 6. Further, ethylene oxide was produced by a similar method as above, except that the catalyst A obtained in Example 1 was used and the inner diameter of the reaction tube was set at 39 mm, and it was referred to as Example 7. According to the following Expression 5 and Expression 6, conversion (Expression 5) and selectivity (Expression 6) in producing ethylene oxide were calculated. The results are shown in the following Table 1.

```
[Expression 5]
Conversion (%) = (Number of moles of reacted ethylene
/ Number of moles of ethylene in feed gas) x 100


[Expression 6]
Selectivity (%) = (Number of moles of ethylene converted


to ethylene oxide / Number of moles of reacted ethylene) x
100
```

Table 1

| | | | Example | | | | | | | Control | | | | | | | | | |
|---|---|---|---|---|---|---|---|---|---|---|---|---|---|---|---|---|---|---|---|
| | | | 1 | 2 | 3 | 4 | 5 | 6 | 7 | 1 | 2 | 3 | 4 | 5 | 6 | 7 | 8 | 9 | 10 |
| Carrier No. | | | A | B | C | D | E | A | A | a | b | c | d | e | f | g | h | i | j |
| Reaction tube | Inner Diameter | mm | 35 | 35 | 35 | 35 | 35 | 31 | 39 | 35 | 35 | 35 | 35 | 35 | 35 | 35 | 35 | 35 | 35 |
| | Packing density (reaction tube) | kg/L | 0.81 | 0.82 | 0.86 | 0.73 | 0.84 | 0.76 | 0.82 | 0.77 | 0.86 | 0.81 | 0.91 | 0.72 | 0.89 | 0.75 | 0.42 | 0.87 | 0.54 |
| Catalyst | Bulk density (carrier) | kg/L | 0.74 | 0.70 | 0.79 | 0.61 | 0.73 | 0.74 | 0.74 | 0.70 | 0.74 | 0.66 | 0.82 | 0.65 | 0.81 | 0.68 | 0.37 | 0.79 | 0.35 |
| | Specific surface area | m²/g | 1.54 | 1.61 | 1.53 | 1.68 | 1.59 | 1.54 | 1.54 | 0.72 | 0.80 | 2.46 | 1.73 | 1.68 | 1.75 | 1.71 | 3.23 | 0.61 | 3.58 |
| | Water absorption | % | 38.2 | 39.5 | 39.4 | 52.3 | 26.4 | 38.2 | 38.2 | 38.9 | 39.1 | 43.8 | 42.3 | 40.8 | 41.1 | 39.6 | 68.3 | 7.4 | 73.8 |
| | Outer Diameter | mm | 8.62 | 8.26 | 7.01 | 8.21 | 8.28 | 8.62 | 8.62 | 8.63 | 6.91 | 8.50 | 8.72 | 8.66 | 8.70 | 8.78 | 10.23 | 8.28 | 8.68 |
| | Inner Diameter | mm | 3.79 | 3.9 | 2.74 | 3.42 | 3.76 | 3.79 | 3.79 | 3.96 | 3.01 | 4.06 | 3.83 | 3.90 | 2.55 | 7.16 | 6.04 | 3.46 | 3.72 |
| | Length | mm | 8.57 | 8.20 | 7.37 | 8.35 | 8.34 | 8.57 | 8.57 | 8.42 | 6.97 | 8.30 | 4.12 | 13.75 | 8.38 | 8.42 | 10.18 | 8.32 | 8.66 |
| | Length/ Outer Diameter | | 0.99 | 0.99 | 1.05 | 1.02 | 1.01 | 0.99 | 0.99 | 0.98 | 1.01 | 0.98 | 0.47 | 1.59 | 0.96 | 0.96 | 1.00 | 1.00 | 1.00 |
| | Inner Diameter/ Outer Diameter | | 0.440 | 0.472 | 0.391 | 0.417 | 0.454 | 0.440 | 0.440 | 0.459 | 0.436 | 0.478 | 0.439 | 0.450 | 0.293 | 0.815 | 0.590 | 0.418 | 0.429 |
| | Crush strength | N | 64.0 | 78.1 | 82.6 | 50.0 | 100.8 | 64.0 | 64.0 | 74.4 | 63.6 | 32.5 | 75.6 | 72.3 | 88.7 | 29.7 | 25.3 | 156.6 | 12.3 |
| | Wear rate | % | 0.77 | 0.83 | 0.25 | 1.28 | 0.40 | 0.77 | 0.77 | 2.25 | 0.42 | 5.10 | 0.18 | 0.23 | 0.20 | 5.34 | 14.70 | 0.20 | 22.50 |
| Performance | Selectivity | % | 82.6 | 82.4 | 82.3 | 82.0 | 81.5 | 82.5 | 82.6 | 80.7 | 80.7 | 80.3 | 80.3 | 80.6 | 80.4 | 80.3 | 79.8 | 79.5 | - |
| | Conversion | % | 10 | 10 | 10 | 10 | 10 | 10 | 10 | 10 | 10 | 10 | 10 | 10 | 10 | 10 | 10 | 10 | - |
| | Reaction temp. | °C | 239 | 240 | 241 | 233 | 242 | 241 | 238 | 238 | 239 | 247 | 240 | 245 | 239 | 247 | 251 | 244 | - |

From the results shown in the above Table 1, according to this invention, the catalyst for the production of ethylene oxide superior in selectivity can be provided. In addition, according to the method for the production of ethylene oxide using the relevant catalyst, production of ethylene oxide in high yield becomes possible.

**Claims**

1. A catalyst for the production of ethylene oxide comprising a catalyst component supported on a carrier comprising α-alumina as a main component, having a crush strength of at least 40 N, a specific surface area of at least 1.0 m$^2$/g, and a geometrical shape of a hollow cylinder, **characterized in that**;
the catalyst is used by packing in a reaction tube with an inner diameter of at least 28 mm, of a reactor for producing ethylene oxide; and
a length/outer diameter ratio in the hollow cylinder is from 0.5 to 1.5, and an inner diameter/outer diameter ratio in the hollow cylinder is within a range of from 0.38 to 0.80.

2. The catalyst for the production of ethylene oxide according to claim 1, wherein the reactor for producing ethylene oxide is a multi-tubular reactor provided with a plurality of reaction tubes.

3. The catalyst for the production of ethylene oxide according to claim 1 or 2, wherein the catalyst is packed in the reaction tube in a packing density of at least 0.5 kg/L.

4. The catalyst for the production of ethylene oxide according to any one of claims 1 to 3, wherein a water absorption of the carrier is 10 to 70%.

5. A method for the production of ethylene oxide which comprises vapor phase oxidization of ethylene with a molecular oxygen-containing gas in the presence of the catalyst for the production of ethylene oxide set forth in any one of claims 1 to 4.

<table>
<tr><td colspan="2"><strong>INTERNATIONAL SEARCH REPORT</strong></td><td>International application No.<br>PCT/JP2009/055323</td></tr>
</table>

| A. CLASSIFICATION OF SUBJECT MATTER |
| --- |
| *B01J23/66*(2006.01)i, *B01J35/02*(2006.01)i, *B01J35/10*(2006.01)i, *C07D301/08*(2006.01)i, *C07D303/00*(2006.01)i |

According to International Patent Classification (IPC) or to both national classification and IPC

| B. FIELDS SEARCHED |
| --- |
| Minimum documentation searched (classification system followed by classification symbols)<br>B01J21/00-38/74 |

Documentation searched other than minimum documentation to the extent that such documents are included in the fields searched

| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| X | WO 2006/102189 A1 (SHELL OIL CO.),<br>28 September, 2006 (28.09.06),<br>Claims 1, 5 to 7, 10; page 5, lines 15 to 18;<br>page 5, lines 25 to 26; page 14, lines 28 to 32;<br>page 15, lines 12 to 13<br>& JP 2008-534501 A    & EP 1861196 A1 | 1-5 |
| X | JP 2-56246 A (Tsuonguoshiyofuagontsuongonshi),<br>26 February, 1990 (26.02.90),<br>Page 4, lower right column, 3rd line from the<br>bottom to page 5, upper left column, line 4;<br>example 1<br>& EP 327356 A1        & US 5063195 A | 1-5 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>08 June, 2009 (08.06.09) | Date of mailing of the international search report<br>16 June, 2009 (16.06.09) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2009/055323

C (Continuation).    DOCUMENTS CONSIDERED TO BE RELEVANT

| Category* | Citation of document, with indication, where appropriate, of the relevant passages | Relevant to claim No. |
|:---:|---|:---:|
| X | JP 5-329368 A  (Nippon Shokubai Co., Ltd.),<br>14 December, 1993 (14.12.93),<br>Par. Nos. [0045], [0052]<br>& US 5395812 A        & EP 558346 A1 | 1-5 |
| A | JP 11-244699 A  (Nippon Shokubai Co., Ltd.),<br>14 September, 1999 (14.09.99),<br>Par. Nos. [0025], [0026]<br>& US 6153556 A          & US 6281370 B1<br>& EP 927575 A1 | 1-5 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007500596 T
- JP 5329368 A
- JP 2001062291 A
- JP 2002136868 A
- JP 2983740 B
- JP 3256237 B
- JP 3295433 B

**Non-patent literature cited in the description**

- Property of porous substances and application technology thereof. Fujitec Corporation, 1999